# EUROPÄISCHE PATENTSCHRIFT

(11) **EP 3 046 623 B1**
(45) Veröffentlichungstag und Bekanntmachung des Hinweises auf die Patenterteilung: **18.07.2018**
(21) Anmeldenummer: 14771553.6
(22) Anmeldetag: 18.09.2014
(51) Int. Cl.: A61N 5/10, A61M 25/10, A61K 51/12

(54) **APPLIKATOR UND DEPOT FÜR EINE RADIOAKTIVE STRAHLENQUELLE ZUR BRACHYTHERAPIE**
APPLICATOR AND RECEPTACLE FOR A RADIOACTIVE SOURCE FOR BRACHYTHERAPY
APPLICATEUR ET DÉPÔT POUR SOURCE DE RAYONNEMENT RADIOACTIF DESTINÉE À LA BRACHYTHÉRAPIE

(30) Priorität: 19.09.2013 DE 102013110375
(43) Veröffentlichungstag der Anmeldung: 27.07.2016
(73) Patentinhaber: Friebe, Michael, 45657 Recklinghausen (DE)
(72) Erfinder: FRIEBE, Michael, 45657 Recklinghausen (DE); NAVAB, Nassir, 81247 München (DE)
(74) Vertreter: Zimmermann & Partner Patentanwälte mbB
(86) Internationale Anmeldenummer: PCT/EP2014/069929
(87) Internationale Veröffentlichungsnummer: WO 2015/040129

(56) Entgegenhaltungen:
- WO-A1-92/22350
- WO-A1-99/12609
- DE-C- 944 988
- US-A- 3 324 847
- US-A1- 2009 054 721
- US-A1- 2009 209 805
- US-A1- 2012 172 651

## Beschreibung

Die Erfindung liegt auf dem Gebiet medizinischer Gerätetechnik für die ortsgenaue interne Bestrahlung von Tumoren oder temporären postoperativen Kavitäten. Insbesondere betrifft die Erfindung einen Applikator in Form eines Ballons für eine Strahlenquelle ionisierender Strahlung, der eine exakt dosierte Bestrahlung im betreffenden Gewebe ermöglicht.

Die externe Bestrahlung entarteten Gewebes zu therapeutischen Zwecken lässt sich zumeist nicht allein auf das jeweilige Zielgebiet begrenzen, da zwischen Tumor und Strahlenquelle befindliche Schichten gesunden Gewebes zwangsläufig durchstrahlt werden. Dabei wird gesundes Gewebe unnötig belastet. Im Gegensatz hierzu bieten verschiedene Ansätze der internen Strahlentherapie (Brachytherapie) eine Möglichkeit, die unerwünschte Strahlenbelastung gesunden Gewebes einzuschränken, indem der Abstand zwischen der Strahlenquelle und dem Zielgewebe verringert wird. Ebenso kann die therapeutische Wirkung durch die ortsgenaue Platzierung der Strahlenquelle optimiert werden. Insbesondere für die effektive Anwendung von Strahlung mit geringer Eindringtiefe, wie beispielsweise im Fall von α-Strahlern wie Uran und Thorium oder von β⁻-Strahlern wie beispielsweise ⁹⁰Sr, ¹⁰⁶Ru, ^{186/188}Re kann zum Zweck einer genau einstellbaren Strahlendosis ein unmittelbarer Kontakt des Depots mit dem Zielgewebe erwünscht sein. Dieser wird in der Praxis der internen Brachytherapie aber beispielsweise durch sich ansammelndes "Wundwasser" erschwert, sodass eine genaue Dosierung auf Grund unklarer Expositionsverhältnisse verhindert wird. Zudem erlaubt eine derartige Ausführung auch die Bestrahlung von Operationshöhlen, die durch operative Entfernung von Tumorgewebe entstanden sind.

US 2012/172651 A1 lehrt eine Vorrichtung und ein Verfahren zur Bereitstellung einer Doppelballon-Brust-Brachytherapie-Vorrichtung. WO 99/12609 A1 lehrt eine Vorrichtung zur medizinischen Behandlung durch Bestrahlung. WO 92/22350 A1 lehrt eine Vorrichtung zur Tumorbehandlung. US 2009/054721 A1 lehrt ein Flüssigkeitsstrahlenschutzschild für die Brachytherapie. US 2009/0209805 A1 beschreibt eine Vorrichtung zur Gewebsbestrahlung. US 3 324 847 A beschreibt ein radioaktives Katheter.

Vor diesem Hintergrund wird ein Applikator gemäß Anspruch 1 vorgeschlagen. Der vorgeschlagene als Strahlenquelle wirkender Applikator für die interne Brachytherapie überwindet die vorstehend beschriebenen Nachteile bekannter Vorrichtungen. Weitere Ausführungsformen, Modifikationen und Verbesserungen ergeben sich anhand der folgenden Beschreibung und der beigefügten Ansprüche.

Gemäß einer ersten Ausführungsform wird ein Applikator für eine Strahlenquelle zur internen Brachytherapie vorgeschlagen. Der Applikator umfasst einen Tubus mit einer Tubuswandung, die eine Innen- und eine Außenseite hat. Der Tubus weist eine erste, d.h. im Anwendungsfall vordere oder patientenseitige Öffnung und eine zweite oder hintere, im Anwendungsfall vom Patienten abgewandte, Öffnung auf, wobei eine reversibel expandierbare Membran zumindest an der ersten Öffnung so mit dem Tubus verbunden ist, dass sie einen von der Tubuswandung umschlossenen Innenraum gegenüber einem Außenraum bzw. einer Umgebung abgrenzt. Die Umgebung stellt insbesondere eine Körperhöhle oder eine postoperative Kavität dar, die einer Brachytherapie unterzogen werden soll.

Vorteile ergeben sich aus der gezielten Exposition von Strukturen des Außenraumes mit Hilfe eines im Innenraum oder in der elastischen Membran angeordneten Radionuklids, wobei das Radionuklid durch die expandierbare Membran von der zu exponierenden Struktur isoliert ist. Da die expandierbare Membran hinsichtlich ihrer stofflichen Zusammensetzung und hinsichtlich ihrer Dicke während der Exposition so ausgewählt ist, dass ionisierende Strahlung des Radionuklids sie durchdringt, oder von ihr ausgeht, kann eine Brachytherapie mit Hilfe des beschriebenen Applikators hochgenau vorgenommen werden: Die Bestrahlungsdosis und die Expositionsdauer lassen sich exakt festlegen. Bekannte Radionuklid-Depots für die Brachytherapie gestatten keine derartig genau einstellbare Expositionsdauer.

Gemäß einer weiteren Ausführungsform wird ein Applikator vorgeschlagen, wobei die expandierbare Membran die innere Wandung des Tubus vollständig bedeckt und die vordere oder erste Öffnung des Tubus so verschließt, dass die expandierbare Membran bei einer Beaufschlagung des vom Tubus umschlossenen Innenraums mit hydrostatischem Druck an der vom Patienten abgewandten, hinteren Öffnung an der vorderen, bzw. patientenseitigen Öffnung in Form eines Ballons expandiert wird. Dadurch wird bewirkt, dass die - beispielsweise in Form eines radioaktiven Fluids vorliegende - Strahlenquelle durch eine Wandung des Ballons an der vorderen Öffnung des Tubus gegenüber der Umgebung abgrenzt wird.

Vorteile dieser Ausführungsform ergeben sich vor allem aus der über eine jeweilige Größe des Ballons einstellbaren Kontaktfläche des beispielsweise im Ballon befindlichen Radionuklids mit Strukturen außerhalb des Ballons. Insgesamt bestimmt in erster Linie die Größe der Oberfläche des Ballons die Größe der mittels Brachytherapie behandelten Strukturen im Außenraum. Damit ist die flächenmäßige Ausdehnung eines behandelbaren Areals über die Größe des Ballons, respektive dessen Oberfläche, einstellbar.

Gemäß einer weiteren Ausführungsform des vorgeschlagenen Applikators grenzt die das radioaktive Fluid umschließende expandierbare und elastische Membran das radioaktive Fluid von der Tubuswandung ab.

Daraus ergeben sich Vorteile für die mehrfache Verwendung des Applikators mit unterschiedlichen Radionukliden. Beispielsweise wird ein direkter Kontakt des radioaktiven Fluids mit dem Tubus vollständig unterbunden.

Gemäß einer weiteren Ausführungsform wird ein Applikator vorgeschlagen, wobei der Tubus einen äußeren Durchmesser im Bereich von 0,5 bis 30 mm, insbesondere von 2 bis 10 mm, bevorzugt von 4 bis 8 mm, und eine Wandstärke im Bereich von 0,02 bis 1 mm, insbesondere von 0,05 bis 0,25 mm aufweist.

Vorteile ergeben sich aus dem vergleichsweise geringen Durchmesser einer Öffnung zum Einführen des Applikators, respektive des Tubus in eine der Brachytherapie zu unterziehende Gewebe-Struktur.

Gemäß einer weiteren Ausführungsform wird ein Applikator vorgeschlagen, wobei der Durchmesser des Ballons in einer Umfangsrichtung eine Wert erreicht, der den äußeren Durchmesser des Tubus um das Doppelte bis um das 200-fache, insbesondere um das 3- bis 100-fache, bevorzugt um das 5- bis 50-fache übersteigt.

Vorteile dieser Ausführungsform ergeben sich aus der hohen Flexibilität der elastischen Membran. Sie vermag sich während des Ausdehnens zu ihrer endgültigen Größe den räumlichen Dimensionen der Strukturen im Außenraum so anzupassen, dass ein inniger Kontakt des im Ballon, d.h. im Innenraum des Applikators, bereitgestellten Radionuklids mit dem der Brachytherapie zu unterziehenden Gewebes einstellbar ist. Dazu ist zu bemerken, dass die hier verwendete Bezeichnung "Ballon" nicht impliziert, dass die von der elastischen Membran außerhalb des Tubus umschlossene Masse des vom hinteren Ende des Tubus aus bereitgestellten Radionuklids zwingend eine sphärische Form hat. Selbst wenn eine typische Ballonform im gängigen Sprachgebrauch eine rotationssymmetrische Form darstellen sollte, so nimmt unter den hier geltenden Bedingungen eines in unmittelbaren Kontakt mit einer Kavität in einer Gewebsstruktur tretenden Ballons, der Ballon vorrangig die Form der Kavität an, wobei das Volumen der Kavität mit Hilfe von diagnostischen Systemen bestimmt werden kann und damit auch eine genaue Strahlendosierung möglich ist. Die expandierbare Membran kleidet gewissermaßen eine Höhlung im Außenraum aus und ermöglicht so - über die Barriere der elastischen Membran - den Kontakt der Innenseite der betreffenden Kavität mit dem im ggf. deformierten Ballon oder zumindest in/auf dessen Wandung befindlichen Radionuklid.

Gemäß einer weiteren Ausführungsform wird ein Applikator vorgeschlagen, wobei die expandierbare Membran so ausgewählt ist, dass der an der vorderen Öffnung ausbildbare Ballon reversibel ist, sodass bei einem hydrostatischen Druck im Innenraum gleich oder kleiner als der im Außenraum, der äußere Durchmesser des Tubus einen äußeren Durchmesser des Applikators in Umfangsrichtung bestimmt.

Besondere Vorteile dieser Ausführungsform ergeben sich daraus, dass der Ballon vollständig kollabierbar ist, der Applikator somit wieder durch die kleine zuvor zum Einführen benutzte Öffnung zurückgezogen werden kann.

Gemäß einer weiteren Ausführungsform wird ein Applikator vorgeschlagen, der weiterhin eine im Innenraum angeordnete, jedoch vom Innenraum fluidisch isolierte Röhre umfasst, die in direkten fluidischen Kontakt zum Außenraum gebracht werden kann und es gestattet, im Außenraum einen Unterdruck zu erzeugen, wenn zumindest an der hinteren Öffnung die Außenseite der Tubuswandung fluidisch gegenüber dem Außenraum abgedichtet ist. So kann ein unmittelbarer mechanischer Kontakt zwischen der Membran, beispielsweise einem von der elastischen Membran ausgebildeten Ballon und dem Außenraum verbessert werden.

Vorteile dieser Ausführungsform sind beispielsweise, dass der Ballon so in besonders innigen Kontakt zur Innenfläche einer im Außenraum vorliegenden Kavität gebracht werden kann. Etwaige im Außenraum zunächst vorliegende Fluide können abgesaugt werden und stellen mithin keine zusätzliche Barriere für die vom Radionuklid hinter der elastischen Membran ausgehende ionisierende Strahlung dar.

Gemäß einer weiteren Ausführungsform wird ein Applikator vorgeschlagen, wobei die besagte zumindest teilweise im Innenraum angeordnete Röhre in Umfangsrichtung von der elastischen Membran umgeben ist und auf einer Außenseite der den Ballon ausbildenden Membran in Areale der Membran übergeht, die auf zueinander benachbarten Abschnitten ein unterschiedliches Oberflächenprofil und/oder eine unterschiedlicher Rauheit aufweisen.

Vorteile dieser Ausführungsform bestehen beispielsweise darin, dass nicht nur das unmittelbar an der vorderen Öffnung der Röhre anstehende Fluid über einen Unterdruck abgesaugt werden kann, sondern, dass selbst in Umfangsrichtung an die elastische Membran anliegende Strukturen drainiert werden. Damit wird ein inniger Kontakt der Oberfläche des Ballons mit der inneren Oberfläche der im Außenraum vorliegenden Kavität erzielt.

Gemäß einer weiteren Ausführungsform wird ein Applikator vorgeschlagen, wobei sich die Tubuswandung an ihrer Innenseite und an ihrer Außenseite in ihrer stofflichen Zusammensetzung unterscheiden, und eine von der Außenseite in Richtung zur Innenseite verlaufende Schicht ein Material umfasst, das ionisierende Strahlung zumindest teilweise absorbiert.

Vorteile ergeben sich daraus, dass eine Einwirkung der vom Radionuklid ausgehenden ionisierenden Strahlung ausschließlich auf die im Kontakt mit der elastischen Membran befindlichen Strukturen des Außenraumes gewährleistet ist. Es erfolgt keine Strahlenbelastung von gesundem Gewebe über den zur Einführung des Applikators benutzten Kanal.

Gemäß einer weiteren Ausführungsform wird ein Applikator vorgeschlagen, wobei zwischen Innenseite und Außenseite der Tubuswandung ein Material angeordnet ist, das ionisierende Strahlung zumindest teilweise absorbiert.

Vorteile ergeben sich aus einer vereinfachten Ausführung der Tubuswand. Wird ein geeignetes schichtartig aufgebautes Material benutzt, so kann eine nutzbare Verwendungsdauer des Applikators ohne eine Beeinträchtigung der Oberflächengüte des Tubus maximiert werden.

Gemäß einer weiteren Ausführungsform wird ein Instrument bzw. ein Instrumenten-Kit vorgeschlagen, umfassend: - einen Tubus (10) mit einer Tubuswandung, die eine Innen- und eine Außenseite hat, weiter umfassend eine vordere oder erste und eine hintere oder zweite Öffnung, die miteinander durch die Tubuswandung verbunden sind, wobei die Tubuswandung einen Innenraum umschließt; - eine expandierbare Membran, die zumindest mit der ersten oder vorderen Öffnung des Tubus verbunden ist, sodass die expandierbare Membran den Innenraum gegenüber einem Außenraum abgrenzt; - eine im Innenraum angeordnete, jedoch vom Innenraum fluidisch isolierte Röhre, die in direkten fluidischen Kontakt zum Außenraum gebracht werden kann und es gestattet, im Außenraum einen Unterdruck zu erzeugen, wenn der Tubus zumindest abschnittsweise, bevorzugt zumindest in Richtung zur zweiten oder hinteren Öffnung, fluidisch gegenüber dem Außenraum abgedichtet ist, sodass eine Kontaktfläche zwischen der Membran und dem Außenraum vergrößerbar ist.

Vorteile dieses Kits ergeben sich aus den bereits beschriebenen Vorteilen der Vorrichtung. Insbesondere kann der Applikator anwendungsspezifisch mit beliebigen Radionukliden in beliebiger Formulierung - beispielsweise hinsichtlich einer bevorzugten Dosierung des Radionuklids in einer Trägersubstanz, hinsichtlich einer bevorzugten Viskosität der Formulierung, hinsichtlich einer bevorzugten Temperatur oder einem bevorzugten Volumen des auszubildenden Ballons eingesetzt werden. Beispielhafte Formulierungen des Radionuklids können Flüssigkeiten, Gase, Gele, Emulsionen, Dispersionen, Lösungen oder Pasten sein. Die beschriebene fluidische Abdichtung lässt sich erreichen, wenn der Außenraum zumindest abschnittsweise an der äußeren Wandung des Tubus in Umfangsrichtung anliegt oder - beispielsweise zumindest durch kurzzeitiges mechanisches Anpressen - zum Anliegen gebracht wird.

Gemäß einer weiteren Ausführungsform weist eine Querschnittsfläche der ersten oder vorderen (bei Anwendung patientenseitigen) Öffnung des Tubus eine gleichgroße oder geringere Querschnittsfläche auf, als in Richtung zur oder an der zweiten, im Anwendungsfall vom Patienten abgewandten, Öffnung. Daraus ergibt sich eine konische Form des Tubus, die dessen Einführung in eine Körperöffnung, beispielsweise in eine Wundhöhle, erleichtert. Zusätzlich ermöglicht eine insgesamt konische Form des Tubus eine erleichterte fluidische Abdichtung gegenüber der Körperhöhlung, beispielsweise einer postoperativen Kavität.

Die vorstehend beschriebenen Ausführungsformen können beliebig miteinander kombiniert werden. Dabei können mehrere Ausführungsformen ausgewählt und miteinander kombiniert werden. Ebenso können alle Ausführungsformen unter Weglassen einzelner oder mehrerer spezifischer Merkmale miteinander kombiniert werden.

Gemäß der beschriebenen Ausführungsformen wird der Emitter typischerweise in einen Ballon eingebettet. Der Applikator ermöglicht die Erzeugung eines Unterdrucks an seinem vorderen Ende, wodurch einerseits Wundöffnungen verschlossen und andererseits Gewebsflüssigkeiten abgesaugt werden können. Die beschriebenen Ausführungsformen ermöglichen die Verminderung der Belastung gesunden Gewebes und die vollständige und präzise Dosierung einer Radionuklidexposition des Zielgewebes und überwinden somit eingangs beschriebene Nachteile bekannter Vorrichtungen und Verfahren.

Die beiliegenden Zeichnungen veranschaulichen Ausführungsformen des vorgeschlagenen Applikators und dienen zusammen mit der Beschreibung der Erläuterung der Prinzipien der Erfindung. Die Elemente der Zeichnungen sind relativ zueinander und nicht notwendigerweise maßstabsgetreu dargestellt. Gleiche Bezugszeichen bezeichnen entsprechend ähnliche Teile.
Figur 1 zeigt einen Applikator für eine radioaktive Strahlenquelle zur temporären Brachytherapie;
Fig. 2 zeigt den Aufbau des Applikators mit dem zunächst noch darin enthaltenen Depot gemäß einer Ausführungsform;
Fig. 3 zeigt eine alternative Ausführungsform eines Querschnitts des Applikators, umfassend unterschiedliche Kanäle;
Fig. 4 zeigt eine Anwendung des Applikators zur Brachytherapie beim Mammakarzinom.

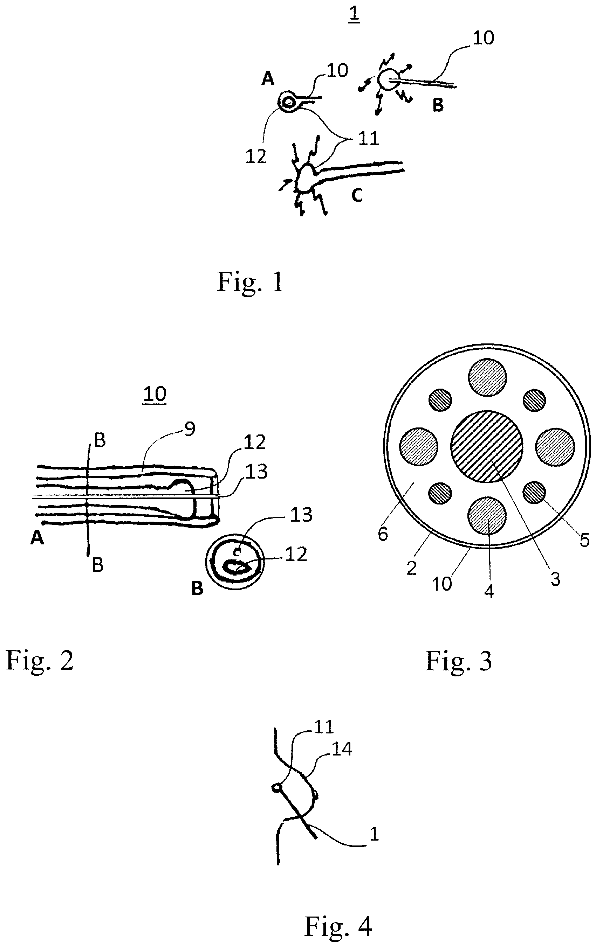

Insbesondere sind in Fig. 1 drei verschiedene Ausführungsformen A, B und C des Applikators 1 in dessen jeweiliger Gestalt während der Brachytherapie skizziert. Der Applikator 1 umfasst typischerweise einen langgestreckten Tubus 10 und eine am vorderen Ende desselben angeordnete ballonartige Erweiterung. Der Ballon umschließt eine Matrix 12, die das für die Kurzzeit-Bestrahlung vorgesehene Radionuklid enthält und verhindert so den unmittelbaren Kontakt des Radionuklids mit der Umgebung. Die Matrix 12 kann beispielsweise in Form einer Lösung, einer Emulsion oder einer Paste vorliegen.

Der Ballon bzw. die ballonartige Erweiterung wird typischerweise durch einen einseitig verschlossenen Schlauch 12, der aus einem Elastomer besteht, gebildet. Der Ballon wird erst bei der Befüllung des Elastomerschlauchs 12 mit der Matrix ausgebildet. Der Ballon bildet sich somit erst beim einseitigen Ausdehnen des Elastomerschlauchs aus. Ähnlich einem nicht aufgeblasenen Luftballon liegt der Elastomerschlauch zunächst im Inneren des Tubus 10 als einseitig verschlossene Kapillare mit elastischer Wandung vor. Am hinteren offenen Ende des Elastomerschlauchs das sich am hinteren Ende des hier ebenso nicht gezeigten Tubus 10 befindet, wird sodann weitere Matrix unter Druck in den Elastomerschlauch gepresst. Das kann durch einen einfachen Kolbenmechanismus, beispielsweise eine Kolbenhubpipette, eine Dosiervorrichtung, beispielsweise einer Dosierpumpe, oder unter Zuhilfenahme einer Spritze mit feststellbarem Kolben erfolgen.

Da die Wandung 9 des Tubus 10 starr ist, widersteht sie dem hydrostatischen Druck der Matrix, sodass die Elastomermembran mit der davon umschlossenen Matrix am vorderen Ende des Tubus ausgestülpt wird und den Ballon ausbildet. So kann durch die Wandung des Ballons hindurch eine Bestrahlung des den Ballon umschließenden Gewebes erfolgen. Wie in Fig. 1B gezeigt, kann die den Ballon ausbildende Membran auch außerhalb des Tubus 10 befestigt sein, die Beschickung des Ballons somit unmittelbar über den Tubus erfolgen. Ebenso kann, wie in Fig. 1C gezeigt, der Applikator einstückig ausgeführt sein. Das den Applikator ausbildende Material weist hier im Bereich des Tubus eine höhere Materialstärke auf, als an dessen vorderem Ende, sodass beim Beaufschlagen der darin befindlichen Matrix der Ballon am vorderen Ende des Tubus ausgebildet wird.

Um den Gewebskontakt des Ballons mit umgebendem Gewebe zu verbessern, kann über eine zusätzliche, vom Ballon fluidisch getrennte Kapillare 13 verwendet werden. Eine beispielhafte Ausführungsform hierzu ist in Fig. 2 gezeigt. Eine im Inneren des Tubus 10 verlaufende Kapillare 13 dient zur Erzeugung eines Unterdrucks am vorderen, bereits in das zu bestrahlende Gewebe eingeführten Tubus 10 des Applikators 1. Beispielsweise kann über die Kapillare 13 Gewebsflüssigkeit bzw. Wundwasser abgepumpt werden, während der Ballon allmählich mit Hilfe eingepresster Matrix mit Radionuklid 12 ausgestülpt wird. Der Ballon bzw. der den Ballon ausbildende einseitig geschlossene Elastomerschlauch ist entsprechend der hier skizzierten Ausführungsform gesondert ausgeführt. Fig. 2A zeigt einen Längsschnitt durch das vordere Ende des Tubus 10. Fig. 2B zeigt einen Querschnitt entlang der in Fig. 2A mit B-B gekennzeichneten Schnittebene. Gemäß bevorzugten Ausführungsformen weist der Tubus zumindest an seinem vorderen, patientenseitigen Endabschnitt eine zusätzliche abschirmende Wandung 9 auf. Diese Abschirmung kann beispielsweise durch ein zumindest am vorderseitigen Endabschnitt in der Wandung 9 eingebettetes oder auf ihrer Innenseite aufgebrachtes Abschirmmittel 2, beispielsweise eine geeignete Absorberschicht 2, gebildet sein. Als geeignetes Absorbermaterial kommen beispielsweise Bleifolie oder eine bleihaltige Legierung, ebenso Wolfram oder wolframhaltige Legierungen oder andere dem Fachmann bekannte Absorbermaterialien in Betracht.

Besagte Materialien können mit verschiedenen dem Fachmann bekannten Techniken auf die Innenseite oder auf die Außenseite oder auf beide Seiten einer metallischen Röhre aufgebracht werden. Ebenso können die - beispielsweise mittels CVD, einer Plasmabehandlung oder mittels Plating oder Sputtertechniken aufgebrachten Schichten abschließend mit einer Passivierungsschicht versehen werden, die einen direkten Kontakt des Absorbermaterials mit der Umgebung verhindert.

Typischerweise ist eine derartige Absorberschicht beidseitig wie auch an ihrem vorderen Rand geeignet oberflächenbeschichtet, um einen Kontakt mit der Umgebung zu unterbinden. Das hat den Vorteil, dass eine Korrosion des Absorbermaterials verhindert wird, kein Kontakt von Schwermetallen mit Gewebe oder Körperflüssigkeiten erfolgt, der Tubus 10 vor mechanischer Beschädigung geschützt ist und den üblichen Sterilisationsprozeduren unterzogen werden kann.

Gemäß einer weiteren Ausführungsform kann die Kapillare 13 ebenso im Inneren des Elastomerschlauchs verlaufen und an dessen vorderen geschlossenen Ende austreten. Wenn die äußere Oberfläche des Elastomerschlauchs an dessen vorderen Ende zusätzliche Strukturen, beispielsweise Erhabenheiten, Furchen oder Riefen aufweist, kann beim Anlegen eines Unterdrucks Wundflüssigkeit nicht nur unmittelbar von der vorderen Öffnung der Kapillare 13 sondern aus der gesamten Umgebung des sich formenden Ballons über dessen gesamte Oberfläche hinweg erfasst und abgesaugt werden. So wird ein besonders inniger Gewebskontakt erreicht. Derartige Erhabenheiten, Furchen und Kanälchen können durch geeignete Partikel-Zusätze zum Elastomer, ebenso aber allein durch eine alternierend unterschiedlich starke und/oder elastische Wandstärke erzeugt werden.

Vorteilhafterweise verhindert der vorgeschlagene Applikator in Form des mit dem Radionuklid beschickten Ballons einerseits den unmittelbaren Gewebskontakt des verwendeten Nuklids. Dadurch wird eine Infiltration des Gewebes durch das Nuklid verhindert. Dennoch wird durch die flexible und dünne Außenwand des ballonförmigen Depots ein weitestgehend inniger Kontakt mit dem zu bestrahlenden Gewebe und so eine effektive Exposition während der Brachytherapie ermöglicht. Zusätzlich verhindert der mit einer abschirmenden Schicht 2 ausgestattete Tubus 10 die unerwünschte Exposition umliegenden gesunden Gewebes. Andererseits ist durch die beschriebenen konstruktiven Merkmale die Entnahme des Depots mit Ablauf der vorgesehenen Behandlungsdauer wesentlich erleichtert. Insgesamt ist somit eine exakt dosierte Bestrahlung auch im Falle komplex geformter Kavitäten im betreffenden Gewebe erreichbar.

Fig. 3 veranschaulicht einen Querschnitt durch einen Applikator gemäß einer alternativen Ausführungsform. Hier ist der Tubus 10 auf der Innenseite mit einem Abschirmmittel 2 beschichtet. Der Tubus 10 des Applikators kann verschiedene Arbeitskanäle aufweisen. Beispielsweise können neben einem durchgehenden Hauptarbeitskanal 3 für den einen Ballon ausbildenden Elastomer-Schlauch bis zu vier zusätzliche durchgehende Arbeitskanäle 4 für Werkzeuge oder Dosiermessungen oder für die zusätzliche diagnostische Bildgebung sowie weiterhin bis zu 6 durchgehende Kanäle 5 für die lokalisierte Erzeugung des gewünschten Vakuums vorgesehen sein.

Fig. 4 veranschaulicht die Anwendung des Applikators 1 mit einem an der ersten Öffnung des Tubus ausgebildeten Ballon 11 im Fall der Brachytherapie eines Mammakarzinoms der weiblichen Brust 14.

Der Applikator kann nach chirurgischer Entnahme eines Tumors eingesetzt werden. Dabei wird die nach dem chirurgischen Eingriff verbliebene Wundöffnung durch den mittels der in Fig. 2 gezeigten Kapillare 13 oder mittels der in Fig. 3 gezeigten Kanäle 5 erzeugten Unterdruck verschlossen.

Gemäß weiteren Ausführungsformen kann die den Ballon ausbildende Membran ein Elastomer umfassen, das selbst auf geeignete Art und Weise mit Radionuklid angereichert ist. Beispielsweise sind dem Fachmann vernetzungsfähige und chelatbildende Verbindungen bekannt, die auf eine Polymermembran aufgetragen und mit jener vernetzt oder in eine elastische Polymermembran eingebracht werden können. Insbesondere können derartige, mit Radionuklid versehene Moleküle unmittelbar in die elastische Membran eingebracht werden oder können zur Ausbildung der Membran selbst dienen. In einem solchen Fall wird der Ballon einfach mit einem radionuklidfreien Fluid aufgeblasen. Gemäß einer Modifikation dieser Ausführungsform kann dieses Fluid zusätzlich temperiert, beispielsweise gekühlt, werden. Das kann für eine während der Brachytherapie verminderte Durchblutung und eine dadurch verminderte Schädigung von Blutplasmazellen von Vorteil sein.

Wenngleich hierin spezifische Ausführungsformen dargestellt und beschrieben worden sind, liegt es im Rahmen der vorliegenden Erfindung, die gezeigten Ausführungsformen geeignet zu modifizieren, ohne vom Schutzbereich der vorliegenden Erfindung abzuweichen. Sie Erfindung ist in den Ansprüchen definiert; andere Ausführungsformen sind jediglich exemplarisch.

## Patentansprüche

1. Applikator (1) für eine Strahlenquelle zur internen Brachytherapie, umfassend:
- einen Tubus (10), umfassend:
- eine Tubuswandung,
- eine erste Öffnung, und
- eine zweite Öffnung; und
- eine expandierbare Membran:
- die ein radioaktives Fluid umschließt und
- die, unter Bildung eines Ballons (11), reversibel aufweitbar ist;
wobei die expandierbare Membran so geformt ist, dass der Ballon (11) mit dem Tubus (10) zumindest an dessen erster, im Anwendungsfall patientenseitiger, Öffnung verbunden ist,
wobei die expandierbare Membran zumindest abschnittsweise mit der Tubuswandung eines Vorderabschnitts des Tubus (10) in mechanischem Kontakt steht, sodass unter Beaufschlagung des radioaktiven Fluids mit hydrostatischem Druck der Ballon (11) an der ersten oder patientenseitigen Öffnung des Tubus (10) aus dem Tubus (10) austreten kann
wobei der Applikator (1) weiterhin umfasst:
- eine im Wesentlichen im Tubus (10) angeordnete, vom Tubus (10) und vom Ballon (11) fluidisch isolierte Röhre mit einer ersten und einer zweiten Öffnung, die in direkten fluidischen Kontakt zu einem Außenraum steht und es gestattet, im Außenraum einen Unterdruck zu erzeugen, wenn der Tubus (10) zumindest abschnittsweise fluidisch gegenüber dem Außenraum abgedichtet ist, sodass eine Kontaktfläche zwischen der Wandung des Ballons (11) und dem Außenraum vergrößerbar ist, wobei die Röhre in Umfangsrichtung von der expandierbaren Membran umgeben ist und auf einer Außenseite der Membran in zueinander benachbarte Areale der Membran übergeht.

2. Applikator nach Anspruch 1, wobei die expandierbare Membran das radioaktive Fluid von der Tubuswandung abgrenzt.

3. Applikator (1) gemäß Anspruch 1 oder 2, wobei der Tubus zumindest in einem an die vordere Öffnung angrenzenden Bereich einen äußeren Durchmesser von 0,5 bis 30 mm, insbesondere von 2 bis 10 mm, bevorzugt von 4 bis 8 mm, und eine Wandstärke von 0,02 bis 1 mm, insbesondere von 0,05 bis 0,25 mm aufweist.

4. Applikator (1) nach einem der vorstehenden Ansprüche, wobei der Ballon (11) in zumindest einer Umfangsrichtung expandierbar ist und den äußeren Durchmesser des Tubus (10) in einem an dessen erste Öffnung angrenzenden Bereich um das Doppelte bis um das 200-fache, insbesondere um das 3- bis 100-fache, bevorzugt um das 5- bis 50-fache übersteigt.

5. Applikator (1) nach Anspruch 1, wobei die zueinander benachbarten Areale der Membran ein unterschiedliches Oberflächenprofil und/oder eine unterschiedlicher Rauheit aufweisen.

6. Applikator (1) nach einem der vorstehenden Ansprüche, wobei sich die Tubuswandung an ihrer Innenseite und an ihrer Außenseite stofflich unterscheidet, wobei eine von der Außenseite in Richtung zur Innenseite verlaufende Schicht ein Material umfasst, das ionisierende Strahlung zumindest teilweise absorbiert.

7. Applikator (1) nach einem der vorstehenden Ansprüche, wobei zwischen Innenseite und Außenseite der Tubuswandung ein Material angeordnet ist, das ionisierende Strahlung zumindest teilweise absorbiert.

## Claims

1. An applicator for a radiation source for internal brachytherapy, comprising:
- a tube (10), comprising:
- a tube wall,
- a first opening, and
- a second opening; and
- an expandable membrane:
- enclosing a radioactive fluid and
- being reversibly expandable while forming a balloon (11);
wherein the membrane is shaped such that the balloon (11) is connected with the tube (10) at least at its first opening, said first opening comprising an patient-side opening when the applicator is in use,
wherein the expandable membrane is at least in some sections in mechanical contact with the tube wall of a front section of the tube (10), so that, when hydrostatic pressure is applied to the radioactive fluid, the balloon can exit from the tube at the tube's first or patient-side opening,
wherein the applicator (1) further comprises:
- a pipe substantially disposed within the tube (10) in fluid isolation from the tube and the balloon and having a first and a second opening being in direct fluid contact to an exterior space and allowing a negative pressure to be generated in the exterior space, when the tube (10) is at least in some sections fluid-sealed with respect to the exterior space so that a contact area between the wall of the balloon (11) and the exterior space is enlargeable, wherein the pipe is surrounded in a circumferential direction by the expandable membrane and on an outer side of the membrane merges in mutually adjacent areas of the membrane.

2. The applicator (1) according to claim 1, wherein the expandable membrane delimits the radioactive fluid from the tube wall.

3. The applicator (1) according to claim 1 or 2, wherein the tube has at least in an area adjacent to the first opening an outer diameter of 0.5 to 30 mm, optionally of 2 to 10 mm, preferentially of 4 to 8 mm, and a wall thickness of 0.02 to 1 mm, optionally of 0.05 to 0.25 mm.

4. The applicator (1) according to any one of previous claims, wherein the balloon (11) is expandable in at least one circumferential direction and exceeds the outer diameter of the tube (10) in an area adjacent to the first opening thereof twice to 200 times, optionally 3 to 100 times, typically 5 to 50 times.

5. The applicator (1) according to claim 1, wherein the adjacent areas of the membrane exhibit a different surface profile and/or a different roughness.

6. The applicator (1) according to any one of previous claims, wherein the tube wall on its inner side and its outer side differs in its material composition, wherein a layer extending from the outer side toward the inner side comprises a material which at least partially absorbs ionized radiation.

7. The applicator (1) according to any one of previous claims, wherein between the tube wall's inner side and outer side a material is disposed which at least partially absorbs ionized radiation.

## Revendications

1. Applicateur (1) pour une source de rayonnement destinée à la brachythérapie interne, comprenant :
- un tube (10), comprenant :
- une paroi de tube,
- une première ouverture, et
- une deuxième ouverture ; et
- une membrane expansible :
- qui enveloppe un fluide radioactif et
- qui peut s'élargir de manière réversible en formant un ballon ;
sachant que la membrane expansible est formée de telle façon que le ballon (11) soit relié au tube (10) au moins au niveau de sa première ouverture, côté patient en cas d'utilisation,
sachant que la membrane expansible est en contact mécanique au moins par section avec la paroi de tube d'une section avant du tube (10) de sorte que le ballon (11) puisse sortir du tube (10) au niveau de la première ouverture ou de l'ouverture côté patient du tube (10) en cas d'application d'une pression hydrostatique sur le fluide radioactif,
sachant que l'applicateur (1) comprend en outre :
- un tuyau essentiellement disposé dans le tube (10), isolé fluidiquement du tube (10) et du ballon (11), présentant une première et une deuxième ouverture, et qui est en contact fluidique direct avec un espace extérieur et permet de générer une dépression dans l'espace extérieur lorsque le tube (10) est étanchéifié fluidiquement au moins par section par rapport à l'espace extérieur, de sorte qu'une surface de contact entre la paroi du ballon (11) et l'espace extérieur puisse s'agrandir, sachant que le tuyau est entouré par la membrane expansible en direction circonférentielle et se prolonge d'un côté extérieur de la membrane en des secteurs adjacents les uns des autres de la membrane.

2. Applicateur selon la revendication 1, sachant que la membrane expansible délimite le fluide radioactif par rapport à la paroi de tube.

3. Applicateur (1) selon la revendication 1 ou 2, sachant que le tube présente, au moins dans une zone contiguë à l'ouverture avant, un diamètre extérieur de 0,5 à 30 mm, en particulier de 2 à 10 mm, de préférence de 4 à 8 mm, et une épaisseur de paroi de 0,02 à 1 mm, en particulier de 0,05 à 0,25 mm.

4. Applicateur (1) selon l'une des revendications précédentes, sachant que le ballon (11) est expansible dans au moins une direction circonférentielle et dépasse le diamètre extérieur du tube (10) dans une zone contiguë à sa première ouverture à raison du double jusqu'à un facteur 200, en particulier d'un facteur 3 à 100, de préférence d'un facteur 5 à 50.

5. Applicateur (1) selon la revendication 1, sachant que les secteurs adjacents les uns aux autres de la membrane présentent un profil de surface différent et/ou une rugosité différente.

6. Applicateur (1) selon l'une des revendications précédentes, sachant que la paroi de tube est différente en termes de matière de son côté intérieur et de son côté extérieur, sachant qu'une couche s'étendant du côté extérieur en direction du côté intérieur comprend un matériau qui absorbe au moins en partie un rayonnement ionisant.

7. Applicateur (1) selon l'une des revendications précédentes, sachant qu'un matériau qui absorbe au moins en partie un rayonnement ionisant est disposé entre le côté intérieur et le côté extérieur de la paroi de tube.
